# EUROPEAN PATENT APPLICATION

(11) **EP 4 194 027 A1**
(43) Date of publication of application: **14.06.2023**
(21) Application number: 21869567.4
(22) Date of filing: 23.08.2021
(51) Int. Cl.: A61M 5/158, A61M 5/142, A61M 5/172

(54) **NEEDLE ASSEMBLY, AND LIQUID MEDICINE INJECTION APPARATUS COMPRISING SAME**

(30) Priority: 16.09.2020 KR 20200119441; 16.09.2020 KR 20200119443; 19.08.2021 KR 20210109695
(71) Applicant: Eoflow Co., Ltd., Seongnam-si, Gyeonggi-do 13605 (KR)
(72) Inventor: KIM, Seungha, Goyang-si Gyeonggi-do 10567 (KR); BANG, Wonkyung, Seoul 08239 (KR)
(74) Representative: Cyrson, Matthew Dominic
(86) International application number: PCT/KR2021/011189
(87) International publication number: WO 2022/059944

(57) **Abstract**

A needle assembly and a medical liquid injection device are provided. The needle assembly includes a needle, a cannula into which the needle is inserted, a first holder supporting the needle, a sleeve in which the first holder is disposed in an inner space thereof, and a second holder supporting the cannula, disposed to face one side of the first holder, and having a first end and a second end extending outward, wherein at least one of the first end and the second end has an cutout part with a part removed by a preset length.

## Description

### TECHNICAL FIELD

The present disclosure relates to a device, and more particularly, to a needle assembly and a medical liquid injection device including the same.

### BACKGROUND ART

In general, a medical liquid injection device such as an insulin injection device is used to inject a medical liquid into a patient's body. Although the medical liquid injection devices are sometimes used by professional medical staff such as doctors or nurses, in most cases, it is used by general public such as the patients themselves or guardians. In the case of diabetic patients, especially pediatric diabetic patients, it is necessary to inject a medical liquid such as insulin into the human body at regular intervals. Therefore, a medical liquid injection device in a patch-type that is used by attaching to human body for a certain period of time is being developed. This medical liquid injection device may be used by being attached to the body such as the patient's abdomen or waist in a patch-type for a certain period of time.

It is necessary to have excellent wearability when the medical liquid injection device is attached to the human body, convenient use, excellent durability, and low power consumption. In particular, since the medical liquid injection device is attached to the patient's skin, the user should be able to simply and conveniently insert a needle or cannula into the patient's skin.

### DESCRIPTION OF EMBODIMENTS

### TECHNICAL PROBLEM

The present disclosure provides a needle assembly and a medical liquid injection device that users may operate simply and precisely.

### TECHNICAL SOLUTION TO PROBLEM

One aspect of the present disclosure provides a needle assembly including a needle, a cannula into which the needle is inserted, a first holder supporting the needle, a sleeve in which the first holder is disposed in an inner space thereof, and a second holder supporting the cannula, disposed to face one side of the first holder, and having a first end and a second end extending outward, wherein at least one of the first end and the second end has an cutout part with a part removed by a preset length.

One aspect of the present disclosure provides a needle assembly including a needle, a holder unit supporting the needle, a first sensor disposed on one side of the holder unit and sensing whether the needle is positioned at an insertion position where the needle is inserted into an object, and a second sensor disposed on the other side of the holder unit and sensing whether the needle is positioned at a reference position that is a position before the needle is inserted into the object.

One aspect of the present disclosure provides a needle assembly including a needle, a holder unit supporting the needle, and a sensing unit connected to the holder unit and sensing a position of the needle in a height direction, wherein the sensing unit includes a base having one surface supported by the holder unit and capable of contraction, and a contact unit disposed on the other surface of the base and having conductivity.

### ADVANTAGEOUS EFFECTS OF DISCLOSURE

A needle assembly and a medical liquid injection device according to the present disclosure may stably insert and fix a cannula to an object. When a first holder moves downward, a second holder also moves downward, the cannula and the needle are inserted into the object, and then the position of the second holder is fixed, so the medical liquid may be stably injected into the object.

The needle assembly and medical liquid injection device according to the present disclosure may simply insert the cannula into the object. Since the second holder has a first end and a second end extending to both sides, and at least one of the first end and the second end is partially removed, deformation of the second holder may be allowed. When the second holder passes through a ledge, since a cutout part allows deformation in a width direction of the first end and the second end, the second holder may simply pass through the ledge and be fixed in position by the ledge.

The needle assembly and medical liquid injection device according to the present disclosure may accurately sense the movement and position of the needle. The first sensor senses a motion of the needle being inserted into the object, and the second sensor senses a motion of the needle retracting to a reference position. The needle assembly and medical liquid injection device according to the present disclosure may sense whether preparation for injecting the medical liquid into the object is safely completed. The needle assembly may sense the insertion of the cannula into the object and the withdrawal of the needle from the cannula.

The needle assembly and medical liquid injection device according to the present disclosure may safely and accurately sense whether the needle is inserted or whether the needle is withdrawn. When the needle is inserted into or withdrawn from the object, a sensor unit may accurately sense the position of the needle by contacting a connection part. In particular, the sensor unit is formed of a material having compressibility and shape restoring force, so that when the sensor unit contacts a pad of the connection part, the sensor unit may be stably contacted with the connection part by being compressed.

### BRIEF DESCRIPTION OF DRAWINGS

FIG. 1 is a block diagram illustrating a medical liquid injection system according to an embodiment of the present disclosure.
FIG. 2 is a perspective view illustrating a medical liquid injection device according to an embodiment of the present disclosure.
FIG. 3 is an exploded perspective view of the medical liquid injection device of FIG. 2.
FIG. 4 is a cross-sectional view taken along line IV-IV in FIG. 2.
FIG. 5 is an exploded perspective view illustrating the needle assembly of FIG. 3.
FIG. 6 is a perspective view illustrating the rear of the needle assembly of FIG. 5.
FIGS. 7A and 7B are perspective views illustrating a second holder of FIG. 5.
FIGS. 8 to 10 are cross-sectional views illustrating a driving operation of the needle assembly of FIG. 5.
FIGS. 11 and 12 are cross-sectional views illustrating an operation in which the second holder of FIG. 5 is mounted.
FIG. 13 is a diagram illustrating an arrangement of sensing unit and connection part.
FIG. 14 is a cross-sectional view of the sensing unit of FIG. 13.
FIG. 15 is a plan view illustrating a contact unit of FIG. 14.
FIGS. 16 and 17 are diagrams illustrating contact between the sensing unit and the connection part of FIG. 13.
FIGS. 18 to 20 are cross-sectional views illustrating another embodiment of the sensing unit of FIG. 14.
FIG. 21 is an exploded perspective view illustrating a needle cover assembly of FIG. 3.

### MODE OF DISCLOSURE

One aspect of the present disclosure provides a needle assembly including a needle, a cannula into which the needle is inserted, a first holder supporting the needle, a sleeve in which the first holder is disposed in an inner space thereof, and a second holder supporting the cannula, disposed to face one side of the first holder, and having a first end and a second end extending outward, wherein at least one of the first end and the second end has an cutout part with a part removed by a preset length.

In addition, at least one of the first end and the second end has an end extending to protrude from an outer circumferential surface of the first holder, and a part of the cutout part may be disposed outside the outer circumferential surface of the first holder in a radial direction.

In addition, another part of the cutout part may be disposed between an inner circumferential surface and the outer circumferential surface of the first holder.

In addition, the first end extends to one side, and may have a first cutout part having a slit shape with an open end.

In addition, the second end extends to the other side, and may have a second cutout part extending in a longitudinal direction and having an opening with a closed end.

In addition, the second holder is formed such that the second end is disposed at a higher position than the first end, and a sensor may be disposed at an end of the second end.

Another aspect of the present disclosure provides a medical liquid injection device including a base body, a housing disposed above the base body, a sleeve mounted to the base body and the housing, a first holder inserted into an inner space of the sleeve and supporting a needle, and a second holder supporting a cannula into which the needle is inserted, disposed to face one side of the first holder, and having a first end and a second end extending outward, wherein at least one of the first end and the second end has a cutout part with a part removed by a preset length.

In addition, at least one of the first end and the second end has an end extending to protrude from an outer circumferential surface of the first holder, and a part of the cutout part may be disposed outside the outer circumferential surface of the first holder in a radial direction.

In addition, at least one of the first end and the second end has a locking protrusion protruding from an outside of the cutout part, and the base body may have a ledge supporting the locking protrusion when the second holder descends.

In addition, the first end extends to one side, and may have a first cutout part having a slit shape with an open end.

In addition, the second end extends to the other side, and may have a second cutout part extending in a longitudinal direction and having an opening with a closed end.

In addition, the second holder is formed such that the second end is disposed at a higher position than the first end, a sensor is disposed at an end of the second end, and the sensor may contact one side of the base body when the second holder descends.

Another aspect of the present disclosure provides a needle assembly including a needle, a holder unit supporting the needle, a first sensor disposed on one side of the holder unit and sensing whether the needle is positioned at an insertion position where the needle is inserted into an object, and a second sensor disposed on the other side of the holder unit and sensing whether the needle is positioned at a reference position that is a position before the needle is inserted into the object.

In addition, the second sensor may sense whether the needle is withdrawn from the insertion position and positioned again at the reference position.

In addition, the needle assembly further includes a cannula into which the needle is inserted, and the holder unit may include a first holder supporting the needle and having the second sensor disposed thereon, and a second holder supporting the cannula and having the first sensor disposed thereon.

In addition, the needle assembly may further include a control module recognizing that the needle is withdrawn from the object while the cannula is inserted into the object when both the first sensor and the second sensor are sensed.

Another aspect of the present disclosure provides a medical liquid injection device including a base, a housing disposed above the base, and a needle assembly inserted into the housing, wherein the needle assembly includes a needle, a holder unit supporting the needle, a first sensor disposed on one side of the holder unit and sensing whether the needle is positioned at an insertion position where the needle is inserted into an object, and a second sensor disposed on the other side of the holder unit and sensing whether the needle is positioned at a reference position that a position before the needle is inserted into the object.

In addition, the second sensor may sense whether the needle is withdrawn from the insertion position and positioned again at the reference position.

In addition, the needle assembly may further include a cannula into which the needle is inserted, and the holder unit may include a first holder supporting the needle and having the second sensor disposed thereon, and a second holder supporting the cannula and having the first sensor disposed thereon.

In addition, the needle assembly may further include a control module recognizing that the needle is withdrawn from the object while the cannula is inserted into the object when both the first sensor and the second sensor are sensed.

Another aspect of the present disclosure provides a needle assembly including a needle, a holder unit supporting the needle, and a sensing unit connected to the holder unit and sensing a position of the needle in a height direction, wherein the sensing unit includes a base whose one surface is supported by the holder unit and capable of contraction, and a contact unit disposed on the other side of the base and having conductivity.

In addition, when the needle is inserted into an object, the contact unit contacts a connection part and is electrically connected to the connection part, and the base may be compressed in a height direction when the contact unit contacts the connection part.

In addition, the contact unit may be woven so that a first conductive member and the second conductive member cross each other.

In addition, the contact unit may have a preset roughness or protrusion on a surface exposed to an outside.

Also, the sensing unit may include a first sensor disposed on one side of the holder unit and sensing whether the needle is positioned at an insertion position where the needle is inserted into an object.

Also, the sensing unit may include a second sensor disposed on the other side of the holder unit and sensing whether the needle is positioned at a reference position that is a position before the needle is inserted into an object.

Another aspect of the present disclosure includes a base; a housing disposed above the base, a needle assembly disposed inside the housing, and a connection part disposed adjacent to the needle assembly and having a pair of pads, wherein the needle assembly may include a needle, a holder unit supporting the needle, and a sensing unit supported by the holder unit and disposed to face the connection part.

In addition, the sensing unit may be included a base whose one surface is supported on the holder unit and capable of contraction, and a contact unit disposed on the other side of the base and having conductivity.

In addition, the contact unit may be woven so that a first conductive member and the second conductive member cross each other.

In addition, the contact unit may have a preset roughness or protrusion on a surface exposed to an outside.

Also, when the needle is inserted into an object, the sensing unit may contact the connection part to electrically connect the pair of pads.

### MODE FOR INVENTION

Since the present disclosure may apply various transformations and may have various embodiments, specific embodiments are illustrated in the drawings and described in detail in the detailed description. Effects and characteristics of the present disclosure, and methods for achieving them will become clear with reference to the embodiments described later in detail together with the drawings. However, the present disclosure is not limited to the embodiments disclosed below and may be implemented in various forms.

Hereinafter, embodiments of the present disclosure will be described in detail with reference to the accompanying drawings, and when describing with reference to the drawings, the same or corresponding components are given the same reference numerals, and overlapping descriptions thereof will be omitted.

In the following examples, the singular expression includes the plural expression unless the context clearly dictates otherwise.

In the following embodiments, terms such as include or have means that the features or components described in the specification are present, and the possibility that one or more other features or components will be added is not excluded in advance.

In cases where certain embodiments may be implemented otherwise, a specific process sequence may be performed different from the described sequence. For example, two processes described in succession may be performed substantially simultaneously, or may be performed in an order opposite to the order described.

In the drawings, the size of the components may be exaggerated or reduced for convenience of description. For example, since the size and thickness of each component shown in the drawings are arbitrarily shown for convenience of description, the following embodiments are not necessarily limited to those illustrated.

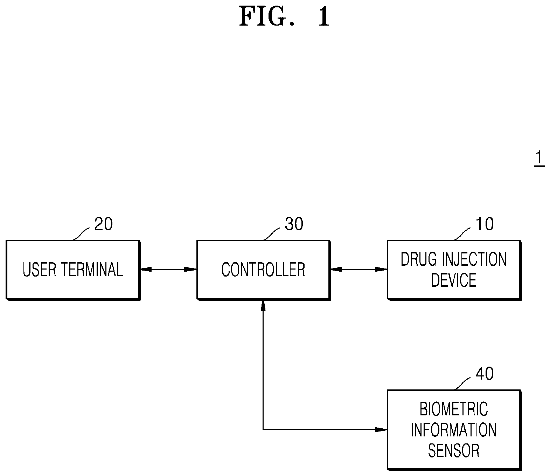

FIG. 1 is a block diagram illustrating a medical liquid injection system 1 according to an embodiment of the present disclosure.

Referring to FIG. 1, a medical liquid injection system 1 may include a medical liquid injection device 10, a user terminal 20, a controller 30 and a biometric information sensor 40. In the medical liquid injection system 1, a user may drive and control the system by using the user terminal 20, and the medical liquid injection device 10 may periodically inject medical liquids based on blood glucose information monitored by the biometric information sensor 40.

The medical liquid injection device 10 injects medical liquids, such as insulin, glucagon, anesthetics, painkillers, dopamine, growth hormone, and smoking cessation aids to be injected into the user based on the data sensed by the biometric information sensor 40.

In addition, the medical liquid injection device 10 may transmit a device status message including remaining battery capacity information of the device, whether the device boots successfully, whether injection is successful, etc. to the controller 30. Messages transmitted to the controller may be transmitted to the user terminal 20 via the controller 30. Alternatively, the controller 30 may transfer improved data obtained by processing received messages to the user terminal 20.

In one embodiment, the medical liquid injection device 10 is provided separately from the biometric information sensor 40 and may be installed apart from an object. In another embodiment, the medical liquid injection device 10 and the biometric information sensor 40 may be provided in one device.

In one embodiment, the medical liquid injection device 10 may be mounted on a user's body. In another embodiment, the medical liquid injection device 10 may be mounted on an animal to inject a medical liquid.

The user terminal 20 may receive an input signal from the user to drive and control the medical liquid injection system 1. The user terminal 20 may generate a signal driving the controller 30 and control the controller 30 to drive the medical liquid injection device 10. In addition, the user terminal 20 may display biometric information measured from the biometric information sensor 40 and may display state information of the medical liquid injection device 10.

The user terminal 20 refers to a communication terminal usable in a wired/wireless communication environment. For example, the user terminal 20 may be a smart phone, tablet PC, PC, smart TV, mobile phone, personal digital assistant (PDA), laptop, media player, micro server, global positioning system (GPS) device, e-book reader, digital broadcasting terminal, navigation, kiosk, MP3 player, digital camera, consumer electronic, device with a camera, and other mobile or non-mobile computing device. In addition, the user terminal 2 may be a wearable device having a communication function and data processing function, such as a watch, glasses, a hair band, and a ring. However, as described above, a terminal equipped with an application capable of Internet communication may be borrowed without limitation.

The user terminal 20 may be connected one-to-one with the pre-registered controller 30. The user terminal 20 may be encrypted and connected to the controller 30 in order to prevent the controller 30 from being driven and controlled by an external device.

In one embodiment, the user terminal 20 and the controller 30 may be separated and provided as separate devices. For example, the controller 30 may be provided to a subject equipped with the medical liquid injection device 10, and the user terminal 20 may be provided to the subject or a third party. The safety of the medical liquid injection system 1 may be increased by driving the user terminal 20 by a guardian.

In another embodiment, the user terminal 20 and the controller 30 may be provided as one device. The controller 30 provided as one with the user terminal 20 communicates with the medical liquid injection device 10 to control medical liquid injection.

The controller 30 transmits and receives data to and from the medical liquid injection device 10, transmits control signals related to injection of medical liquids such as insulin to the medical liquid injection device 10, and may receive control signals related to measurement of biological values such as blood glucose from the biometric information sensor 40.

For example, the controller 30 may transmit an instruction request to measure the user's current state to the medical liquid injection device 10 and receive measurement data from the medical liquid injection device 10 in response to the instruction request.

The biometric information sensor 40 may measure biological values such as blood glucose level, blood pressure, and heart rate of the user according to the purpose. Data measured by the biometric information sensor 40 may be transmitted to the controller 30, and a medical liquid cycle and/or injection amount may be set based on the measured data. Data measured by the biometric information sensor 40 may be transmitted to the user terminal 20 and displayed.

For example, the biometric information sensor 40 may be a sensor for measuring the amount of blood glucose of the object. The biometric information sensor may be a continuous glucose monitoring (CGM) sensor. The continuous glucose monitoring sensor is attached to the object and may continuously monitor the amount of blood glucose.

The user terminal 20, controller 30, and medical liquid injection device 10 may perform communication by using a network. For example, the network may include a local area network (LAN), a wide area network (WAN), a value added network (VAN), a mobile radio communication network, a satellite communication network, and these mutual combinations, may be a data communication network in a comprehensive sense that enables each network component to communicate smoothly with each other, and may include wired internet, wireless Internet and mobile wireless communication network. In addition, wireless communication includes, for example, Wi-Fi, Bluetooth, Bluetooth low energy, Zigbee, Wi-Fi Direct (WFD), ultra wideband (UWB), infrared Data Association (IrDA), Near Field Communication (NFC), 5G, etc. may exist, but are not limited thereto.

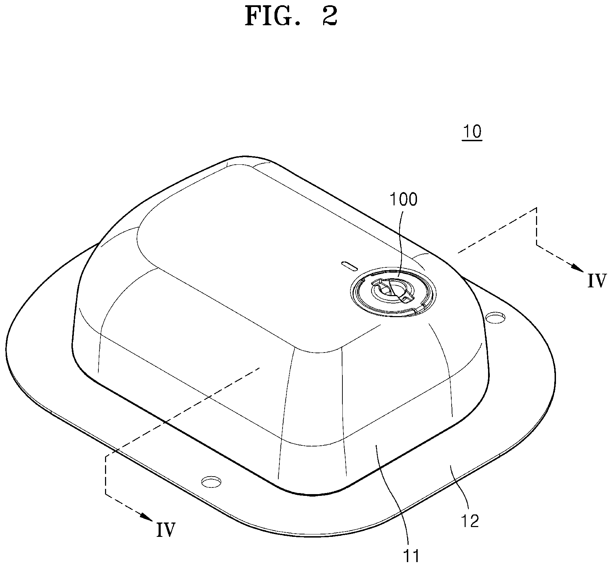

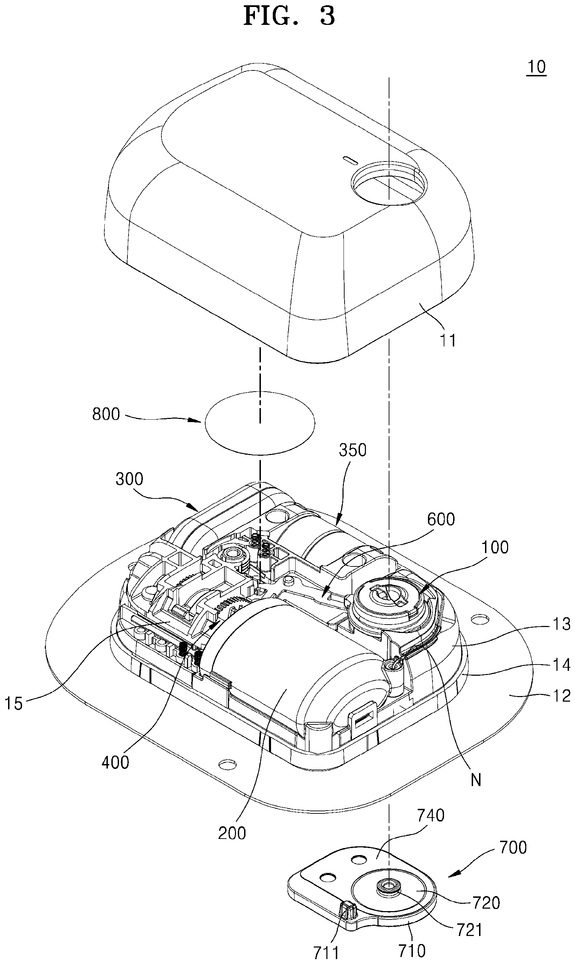

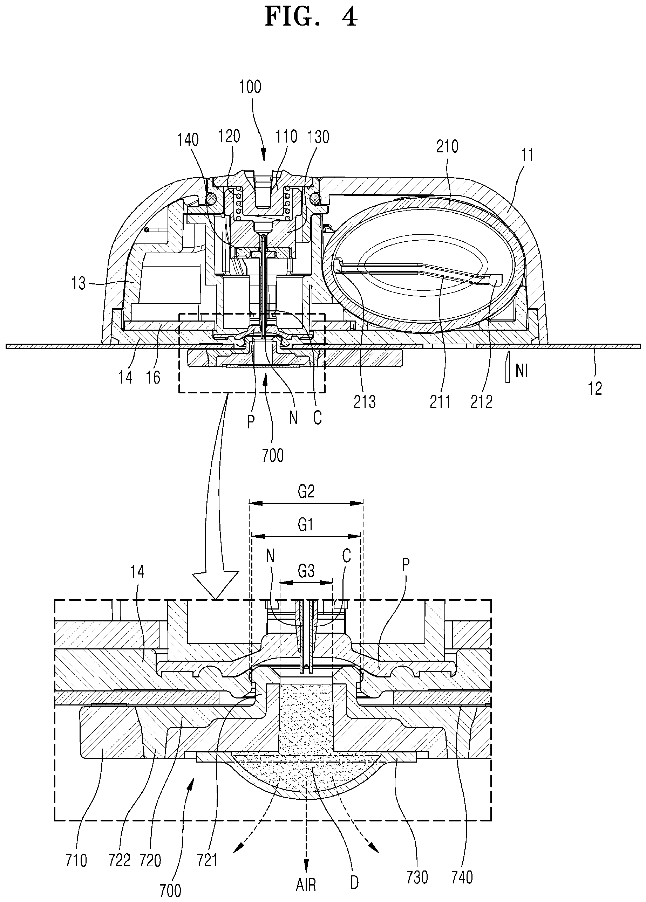

FIG. 2 is a perspective view illustrating a medical liquid injection device 10 according to an embodiment of the present disclosure, FIG. 3 is an exploded perspective view of the medical liquid injection device 10 of FIG. 2, and FIG. 4 is a cross-sectional view taken along line IV-IV in FIG. 2.

Referring to FIGS. 2 to 4, the medical liquid injection device 10 is attached to a user to inject a medical liquid, and may inject the medical liquid stored therein in a set amount to the user.

The medical liquid injection device 10 may be used for various purposes depending on the type of medical liquid to be injected. For example, the medical liquid may include insulin-based medical liquids for diabetics, other pancreas medical liquids, heart medical liquids, and other various types of medical liquids.

One embodiment of the medical liquid injection device 10 may include a housing 11 covering an outside and an attachment part 12 positioned adjacent to the user's skin. The medical liquid injection device 10 includes a plurality of parts disposed in an inner space between the housing 11 and the attachment part 12. A separate bonding unit may be further disposed between the attachment part 12 and the user's skin, and the medical liquid injection device 10 may be fixed to the skin by the bonding unit.

The medical liquid injection device 10 may include a needle assembly 100, a reservoir unit 200, a driving module 300, a battery 350, a driving unit 400, a clutch unit 500, a trigger member 600, a needle cover assembly 700, an alarm unit 800 and a plurality of sensor units.

In the medical liquid injection device 10, the base body may form a frame in which at least one body supports internal parts. The base body may have a first body 13, a second body 14, and a third body 15 according to the arrangement.

The first body 13 is disposed under the housing 11, and the needle assembly 100, the reservoir unit 200, the driving module 300, the battery 350, etc. may be supported in each opening or groove. The second body 14 is disposed below the first body 13 and may be connected to the attachment part 12. The second body 14 may cover a lower portion of the medical liquid injection device 10. The third body 15 may be disposed above the first body 13, and the reservoir unit 200, the driving module 300, the battery 350, the driving unit 400, etc. may be supported in each opening or groove. In the drawing, the first body 13, the second body 14, and the third body 15 are illustrated, but are not limited thereto and may be integrally provided or provided in plurality.

A control module may be disposed inside the medical liquid injection device 10. A control module 16, which is a circuit board, is disposed below the second body 14, and the control module may control the overall operation of the medical liquid injection device 10. The control module 16 is electrically connected to the driving module 300, the battery 350, the alarm unit 800 and a plurality of sensor units to control their driving.

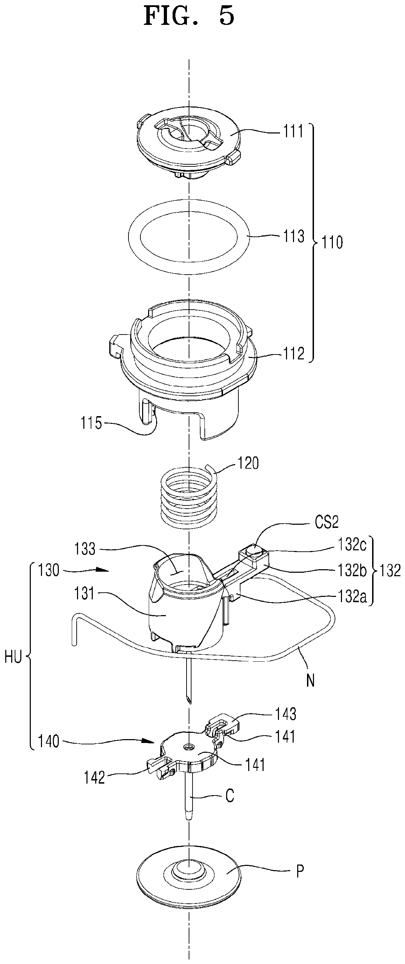

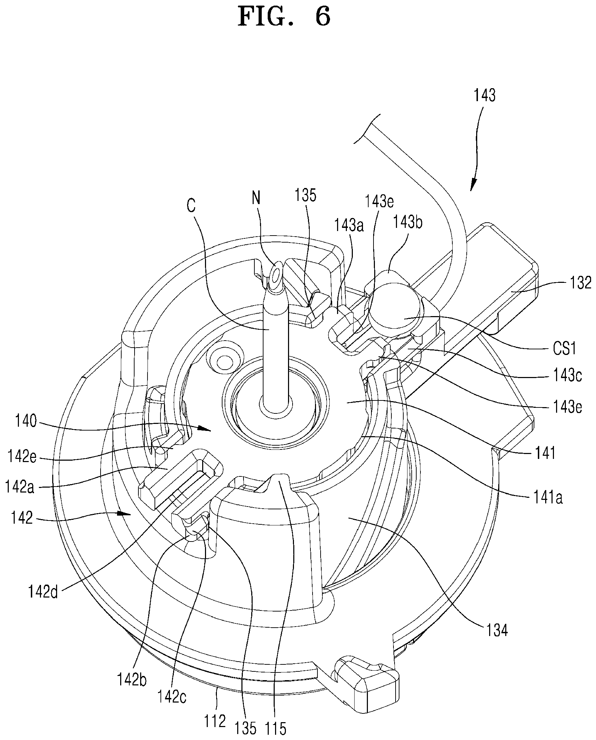

FIG. 5 is an exploded perspective view illustrating the needle assembly 100 of FIG. 3, and FIG. 6 is a perspective view illustrating the rear of the needle assembly 100 of FIG. 5.

Referring to FIGS. 3 to 6, in the medical liquid injection device 10, the user may simply rotate the needle assembly 100 to insert the cannula into the object and start medical liquid injection.

The needle assembly 100 may be mounted on the first body 13. In the needle assembly 100, a needle N and/or a cannula C may move in an axial direction by rotation of a sleeve 110. The needle assembly 100 may include the sleeve 110, an elastic member 120, first holder 130 and second holder 140 which are holder unit HU, the needle N, the cannula C and a patch P.

The sleeve 110 forms an outer appearance of the needle assembly 100 and may rotate about a longitudinal directional axis as a central axis. The sleeve 110 may include a knob 111, a rotating body 112, and a support ring 113.

The knob 111 may be mounted on top of the rotating body 112 and may have a groove on a top surface. The user may rotate the sleeve 110 by manipulating the groove of the knob 111 exposed to an outside. An elastic member 120 is disposed on a lower surface of the knob 111, so that when the elastic member 120 expands in an axial direction, the knob may receive an expansion force.

The rotating body 112 has a substantially cylindrical shape, and an upper opening may be covered by the knob 111. The support ring 113 may be mounted on an outer top of the rotating body 112.

The elastic member 120, the first holder 130 and second holder 140, which are the holder unit HU, may be disposed in an inner space of the rotating body 112. A moving protrusion 115 may be disposed on an inner surface of the rotating body 112. The moving protrusion 115 may move along a guide groove 134 disposed on an outer circumferential surface of the first holder 130. When the rotating body 112 rotates, the moving protrusion 115 moves along side walls of the guide groove 134, so that the first holder 130 may be raised or lowered.

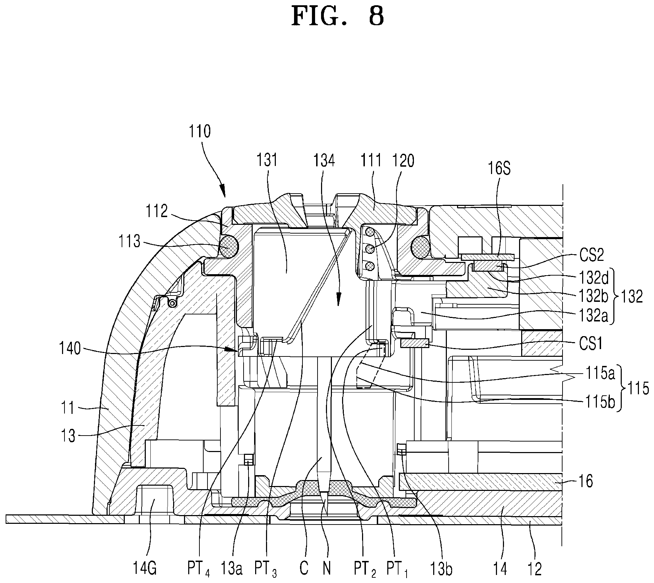

Referring to FIG. 8, the moving protrusion 115 may include an inclined wall 115a and a vertical wall 115b. The inclined wall 115a ensures stable contact when descending along the guide groove 134, and the vertical wall 115b contacts one side of the first holder 130 to limit the moving path of the moving protrusion 115.

The elastic member 120 may be disposed between the sleeve 110 and the first holder 130. When the elastic member 120 expands, the first holder 130 may be moved downward. When the first holder 130 moves upward, the elastic member 120 may be compressed.

The elastic member 120 is not limited to a specific member, and may be made of various members that transmit elastic force in a longitudinal direction. However, in the following description, for convenience of description, the elastic member 120 will be mainly described in a form of spring.

The holder unit HU may support needle N. In one embodiment, the holder unit HU may include the first holder 130 and the second holder 140, and the needle N and the cannula C may be mounted, respectively. In another embodiment, the holder unit HU is provided in one so that only needle N may be mounted.

The first holder 130 may support the needle N. Since the needle N is inserted and fixed to one side of the first holder 130, when the first holder 130 moves in the axial direction, the needle N also moves. The first holder 130 is placed in the inner space of the sleeve 110, and the elastic member 120 is disposed above the first folder.

The first holder 130 may include a first main body 131 , a connection block 132 , a first space 133 and the guide groove 134. The first main body 131 is inserted into the rotating body 112 of the sleeve 110, and the first space 133 in which the elastic member 120 is disposed is formed inside.

The connection block 132 extends from one side of the first main body 131 and may support the needle N. The connection block 132 may have a reinforcing end 132a, an extension end 132b, a slit 132c, and a first seating groove 132d.

The reinforcing end 132a is connected to the first main body 131 and may have a predetermined thickness. The needle N is inserted into the reinforcing end 132a, and the position of the needle N may be fixed. A second end 143 of the second holder 140 may be supported under the reinforcing end 132a. When the elastic member 120 expands, the second holder 140 may be fixed to a second ledge 13b by applying pressure to the second end 143 while the reinforcing end 132a moves downward.

The extension end 132b is connected to the reinforcing end 132a, and the first seating groove 132d is disposed at an end of the extension end. A second sensor CS2 may be inserted into the first seating groove 132d. The second sensor CS2 is disposed above the end of the extension end 132b and is disposed to contact a second connection part of a sub control module 16S.

The slit 132c may be installed in at least one of the reinforcing end 132a and the extension end 132b. The slit 132c may increase a flexibility of the connection block 132. In particular, the slit 132c may maintain a constant contact between the second sensor CS2 and the second connection part of the sub control module 16S by increasing the flexibility of the extension end 132b.

Referring to FIG. 8, the first holder 130 may have the guide groove 134 on the outer surface. The guide groove 134 may guide the movement of the moving protrusion 115. When the sleeve 110 rotates, the moving protrusion 115 moves along a preset path formed by the guide groove 134, so that the first holder 130 may move up or down.

The guide groove 134 may include a first stop groove PT₁ , a first wall PT₂, a second wall PT₃, and a second stop groove PT₄.

The first stop groove PT₁ is a groove where the moving protrusion 115 is initially located, where the elastic member 120 maintains a contracted state.

The first wall PT₂ extends in a longitudinal direction of the first holder 130. When the elastic member 120 expands, the first holder 130 descends, and the moving protrusion 115 may maintain contact with the first wall PT₂. When the first holder 130 moves downward, the first wall PT₂ may guide the movement of the first holder 130.

The second wall PT₃ is extended to have a preset inclination. When the moving protrusion 115 moves along the second wall PT₃, the elastic member 120 contracts again and the first holder 130 rises again.

The moving protrusion 115 is inserted into the second stop groove PT₄, so the first holder 130 may return to the reference position again. At this time, the elastic member 120 maintains the contracted state again.

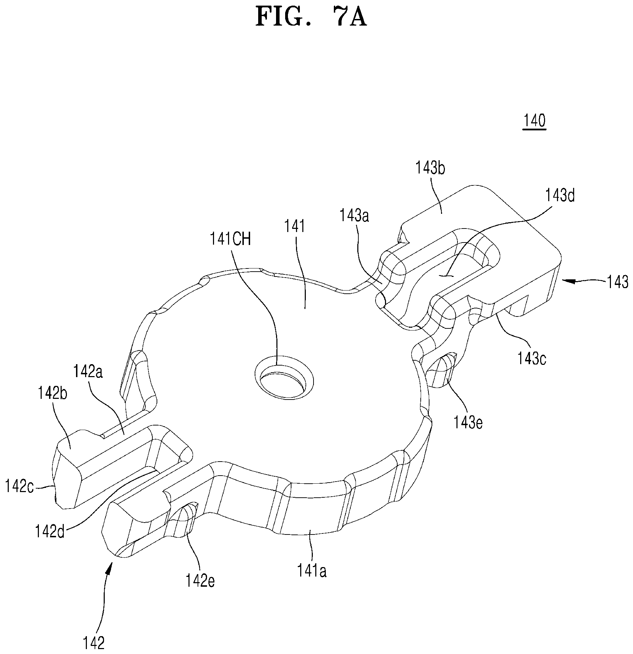

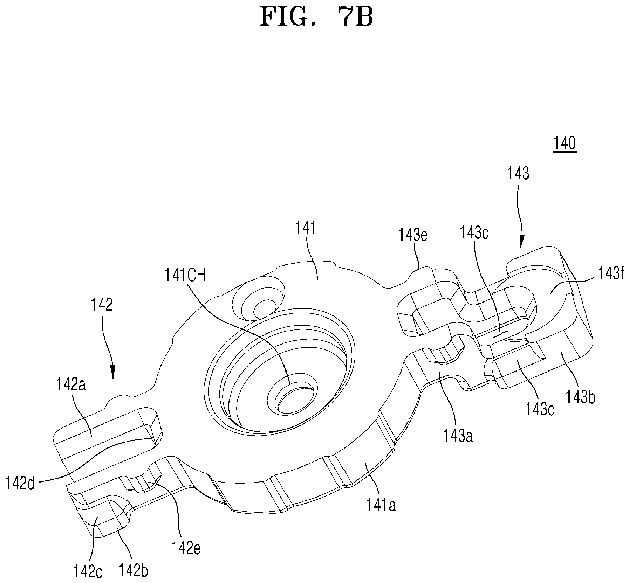

FIGS. 7A and 7B are perspective views illustrating a second holder 140 of FIG. 5.

Referring to FIGS. 5 to 7B, the second holder 140 is disposed to face one side of the first holder 130 and may support the cannula C. The second holder 140 may have a second main body 141, a first end 142 and the second end 143.

The second holder 140 is disposed to face the one side of the first holder 130 and may support the cannula C.

In one embodiment, the second holder 140 may be formed of a material having a predetermined rigidity. The second holder 140 may be deformed by a first cutout part 142d and a second cutout part 143d. When the second holder 140 is inserted into a first ledge 13a and the second ledge 13b, a shape of the second holder 140 may change instantaneously. In particular, shapes of a first locking protrusion 142b and second locking protrusion 143b of the second holder 140 is changed, so that the first locking protrusion 142b may be inserted into the first ledge 13a, and the second locking protrusion 143b may be inserted into the second ledge 13b.

In another embodiment, the second holder 140 is formed of a flexible material, so that its shape may be momentarily deformed when an external force is applied. Flexibility of the second holder 140 may be increased by the first cutout part 142d and the second cutout part 143d.

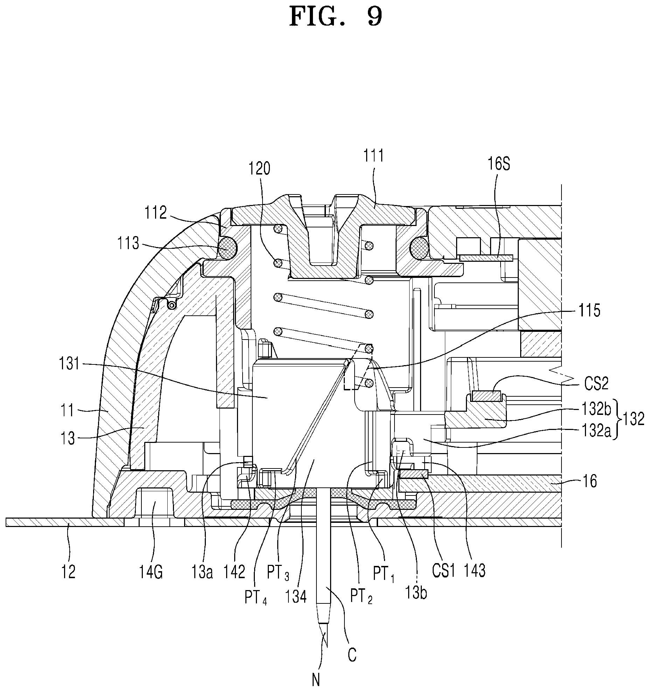

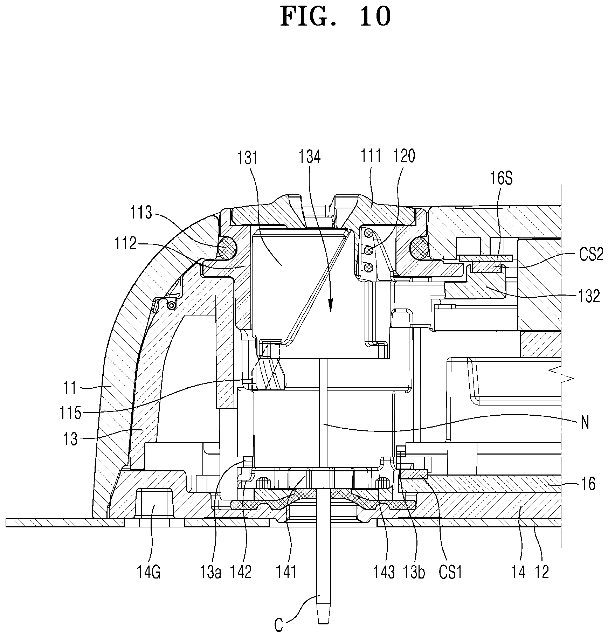

The second holder 140 maintains contact with the first holder 130 at the position illustrated in FIGS. 8 and 9, but is separated from the first holder 130 and fixed to the first body 13 when the elastic member 120 contracts again as illustrated in FIG. 10.

The cannula C is inserted into a center of the second main body 141, and the needle N may be selectively engaged. A central hole 141CH into which the needle N is inserted may be disposed at the center of the second main body 141. Since the central hole 141CH is disposed to face the cannula C, the needle N passing through the central hole 141 CH may be inserted into the cannula C.

A lower surface of the second main body 141 may have a first seating groove to which the cannula C is mounted. The cannula C may be inserted into the first seating groove. A top surface of the second main body 141 may contact a bottom of the first holder 130.

A plurality of alignment protrusions 141a may be disposed outside the second main body 141. The alignment protrusion 141a is arranged to come into contact with an inner circumferential surface of the first holder 130, so that the second holder 140 may be aligned inside the first holder 130.

In the second holder 140, the alignment protrusion 141a contacts the first holder 130, so that shaking of the first holder 130 and the second holder 140 may be reduced. In addition, only the alignment protrusion 141a contacts the inner circumferential surface of the first holder 130, so that frictional force may be reduced. By reducing the contact area between the first holder 130 and the second holder 140, the frictional force between the first holder 130 and the second holder 140 may be reduced, and the second holder 140 may be easily separated from the first holder 130.

The second holder 140 may have the first end 142 and the second end 143 extending outwardly, and at least one of the first end 142 and the second end 143 may have a cutout part removed to a preset length.

A end of at least one of the first end 142 and the second end 143 extends to protrude from an outer circumferential surface of the first holder 130, and a part of the cutout part may be disposed outside the outer circumferential surface of the first holder 130 in a radial direction. Also, another part of the cutout part may be disposed between the inner circumferential surface and the outer circumferential surface of the first holder 130.

In one embodiment, in the second holder 140, cutout parts may be disposed on both the first holder 130 and the second holder 140. In another embodiment, in the second holder 140, the cutout part may be disposed on the first holder 130 or the second holder 140. However, hereinafter, for convenience of description, an embodiment in which cutout parts are disposed in both the first holder 130 and the second holder 140 will be mainly described.

The first end 142 protrudes outward from the second main body 141 and may be formed flexibly. At least a part of the first end 142 is cut along a center so that flexibility may be increased.

The first end 142 may include a first extension tab 142a, the first locking protrusion 142b, a first inclined surface 142c, the first cutout part 142d and a first bump 142e.

The first extension tab 142a extends outward from the second main body 141. Referring to FIG. 6, the first extension tab 142a extends in a radial direction and may be inserted into a guide part 135 in which a part of the first main body 131 of the first holder 130 is cut out. A width of the guide part 135 of the first holder 130 may be set larger than a width of the first extension tab 142a. The guide part 135 comes into contact with the first bump 142e protruding outward of the first extension tab 142a.

A portion of the first extension tab 142a may be inserted into a cut portion of the rotating body 112 of the sleeve 110. Since the cut portion of the rotating body 112 has a predetermined length in a circumferential direction, the first extension tab 142a does not interfere with the rotating body 112 even when the sleeve 110 rotates.

The first locking protrusion 142b may be disposed at an end of the first extension tab 142a. The first locking protrusion 142b may be mounted on the first ledge 13a when the second holder 140 descends.

The first inclined surface 142c may be placed on a side of the first locking protrusion 142b. The first inclined surface 142c that descends downward and faces the center of the first extension tab 142a may be placed on the side of the first locking protrusion 142b. The first inclined surface 142c may guide the first locking protrusion 142b to pass the first ledge 13a.

The first cutout part 142d extends to one side of the first end 142 and may have a shape in which a part of the first extension tab 142a is removed. As an embodiment, as illustrated in the drawing, the first cutout part 142d may have a slit shape with an open end. In another embodiment, although not illustrated in the drawings, the first cutout part 142d may have an opening shape with a closed end. However, in the following description, for convenience of description, the first cutout part 142d of the slit shape with the open end will be mainly described.

The first cutout part 142d may be extended along the longitudinal direction of the first extension tab 142a. The first cutout part 142d may extend along a center of the longitudinal direction of the first extension tab 142a to an end of the first extension tab 142a. Although the figure illustrates that the first cutout part 142d extends along the longitudinal direction of the first extension tab 142a with a constant width, it is not limited thereto, and the width of the first cutout part 142d may change.

The first cutout part 142d may be extended to protrude from the outer circumferential surface of the first holder 130. A part of the first cutout part 142d may be placed outside the outer circumferential surface of the first holder 130 in the radial direction. The first locking protrusion 142b may be disposed outside the part of the first cutout part 142d. In addition, since another part of the first cutout part 142d is inserted into the guide part 135 of the first holder 130, it may be disposed between the inner circumferential surface and the outer circumferential surface of the first holder 130.

The first cutout part 142d may induce deformation of the first extension tab 142a in a width direction. The first locking protrusion 142b is inserted into the first ledge 13a, so that the second holder 140 is fixed. Since the first cutout part 142d protrudes from the outer circumferential surface of the first holder 130 and is placed parallel to the first locking protrusion 142b, the first cutout part 142d may be stably inserted and fixed to the first ledge 13a as the first end 142 is deformed in the width direction.

The first bump 142e protrudes outward from the first extension tab 142a. The first bump 142e may contact the guide part 135 of the first holder 130 to guide the movement of the second holder 140. When the second holder 140 descends, the first bump 142e moves along the guide part 135 of the first holder 130. Since the first bump 142e maintains contact with the guide part 135, shaking of the second holder 140 may be reduced and a linear movement of the second holder 140 may be guided.

The second end 143 may include a second extension tab 143a, the second locking protrusion 143b, a second inclined surface 143c, the second cutout part 143d, a second bump 143e and a second seating groove 143f.

The second extension tab 143a extends outward from the second main body 141. Referring to FIG. 6, the second extension tab 143a is extended in a radial direction and may be inserted into the guide part 135 in which a part of the first main body 131 of the first holder 130 is cut out. The width of the guide part 135 of the first holder 130 may be set larger than a width of the second extension tab 143a. The guide part 135 is in contact with the second bump 143e protruding outward of the second extension tab 143a.

A portion of the second extension tab 143a may be inserted into the cut portion of the rotating body 112 of the sleeve 110. Since the cut portion of the rotating body 112 has the predetermined length in the circumferential direction, the second extension tab 143a does not interfere with the rotating body 112 even when the sleeve 110 rotates.

The second locking protrusion 143b may be disposed at an end of the second extension tab 143a. The second locking protrusion 143b may be mounted on the second ledge 13b when the second holder 140 descends.

The second inclined surface 143c may be placed on a side of the second locking protrusion 143b. The second inclined surface 143c descending downward and facing a center of the second extension tab 143a may be disposed on the side of the second locking protrusion 143b. The second inclined surface 143c may guide the second locking protrusion 143b to pass the second ledge 13b.

The second cutout part 143d extends to the other side of the second end 143 and may have a shape in which a part of the second extension tab 143a is removed. In one embodiment, as illustrated in the drawing, the second cutout part 143d may have an opening with a closed end. In another embodiment, although not illustrated in the drawing, the second cutout part 143d may have a slit shape with an open end. However, hereinafter, for convenience of description, the second cutout part 143d having the opening with the closed end will be mainly described.

The second cutout part 143d may be extended along the longitudinal direction of the second extension tab 143a. The second cutout part 143d may be extended by a predetermined length along a center of the longitudinal direction of the second extension tab 143a. Although the drawing illustrates that the second cutout part 143d extends along the longitudinal direction of the first extension tab 142a with a constant width, it is not limited thereto, and the width of the second cutout part 143d may change. In addition, in the drawings, the second cutout part 143d is illustrated in a long hole shape, but is not limited thereto and may have various shapes.

The second cutout part 143d may be extended to protrude from an outer circumferential surface of the first holder 130. A part of the second cutout part 143d may be placed outside the outer circumferential surface of the first holder 130 in a radial direction. The second locking protrusion 143b may be disposed outside the part of the second cutout part 143d. In addition, since another part of the second cutout part 143d is inserted into the guide part 135 of the second holder 140, it may be disposed between the inner circumferential surface and the outer circumferential surface of the first holder 130.

The second cutout part 143d may induce deformation of the second extension tab 143a in a width direction. The second locking protrusion 143b is inserted into the second ledge 13b, so that the second holder 140 is fixed. Since the second cutout part 143d protrudes from the outer circumferential surface of the first holder 130 and is placed parallel to the second locking protrusion 143b, the second cutout part 143d may be stably inserted and fixed to the second ledge 13b as the second end 143 is deformed in the width direction.

The second bump 143e protrudes outward from the second extension tab 143a. The second bump 143e may contact the guide part 135 of the first holder 130 to guide the movement of the second holder 140. When the second holder 140 descends, the second bump 143e moves along the guide part 135 of the first holder 130. Since the second bump 143e maintains contact with the guide part 135, shaking of the second holder 140 may be reduced and a linear movement of the second holder 140 may be guided.

The second seating groove 143f is disposed in a lower portion of the second end 143, and a first sensor CS1 may be mounted. The second seating groove 143f is formed to be depressed to a predetermined depth, and may provide a space in which the first sensor CS1 is fixed.

A part of the second seating groove 143f may be arranged in the second extension tab 143a, and the other part may be arranged in the second cutout part 143d. The second seating groove 143f is disposed at an end of the second end 143, so that the other part may be disposed overlapping with the second cutout part 143d.

When the second holder 140 descends, the end of the second extension tab 143a is not deformed in the width direction, so that fixing force with the first sensor CS1 is maintained. Therefore, even if the second holder 140 descends and is mounted on the second ledge 13b, the first sensor CS1 may stably maintain a fixed position in the second seating groove 143f.

The first end 142 and the second end 143 may have different heights. The first end 142 and the second end 143 may be arranged to be stepped with respect to the second main body 141. The first end 142 extends to one side at the same height as the second main body 141, but the second end 143 may be disposed at a higher position than the first end 142. The first end 142 and the second end 143 are arranged to have a step difference, and the first sensor CS1 may be arranged in a lower portion of the second end 143.

The first end 142 and the second end 143 may be fixed to the first body 13 according to the movement of the second holder 140. When the elastic member 120 expands, the position of the first end 142 may be fixed to the first ledge 13a, and the position of the second end 143 may be fixed to the second ledge 13b.

The sensing unit may measure a height direction position of the needle N. The sensing unit may measure the position of the holder unit HU and may include the first sensor CS1 and the second sensor CS2.

The first sensor CS1 and the second sensor CS2 may sense the position of the holder unit HU. Hereinafter, the reference position is defined as a position before the needle N is inserted into the object or a position where the needle N is withdrawn from the cannula C and returned to its original position. The insertion position is defined as a position where the needle N is inserted into the object or the cannula C is inserted into the object.

The first sensor CS1 is placed on one side of the holder unit HU and may sense whether the needle N is located at the insertion position where the needle N is inserted into the object. The first sensor CS1 may sense whether the needle N is inserted into the object or the cannula C is inserted into the object.

The first sensor CS1 may be installed in the holder unit HU. The first sensor CS1 is placed on the second holder 140 and may move with the cannula C. The first sensor CS1 may be disposed on the lower surface of the second end 143 of the second holder 140.

The first sensor CS1 may contact a first connection part of a main control module 16. When the first holder 130 moves downward and the needle N is inserted into the object, or when the second holder 140 moves downward, the first sensor CS1 may be electrically connected to the first connection part of the main control module 16. When the contact between the first connection part and the first sensor CS1 is confirmed, the main control module 16 recognizes that the first holder 130 descends normally and the cannula C is inserted into the object.

The second sensor CS2 is placed on the other side of the holder unit HU and may sense whether the needle N is located at the reference position that is the position before the needle N is inserted into the object. The second sensor CS2 may sense whether the needle N is withdrawn from the insertion position and positioned again at the reference position.

The second sensor CS2 may be installed on the holder unit HU. The second sensor CS2 is placed in the first holder 130 and may move together with the needle N. The second sensor CS2 may be placed on the top surface of the connection block 132 of the first holder 130.

The second sensor CS2 may contact the second connection part of the sub control module 16S at the reference position. The sub control module 16S is separated from the main control module 16, but may receive electrical signals. The sub control module 16S may be mounted on the inner surface of the housing 11.

Before the needle assembly 100 operates or when the first holder 130 is withdrawn from an insertion position and returned to the reference position, the second sensor CS2 comes into contact with the second connection part of the sub control module 16S.

As an example, when the contact between the second connection part and the second sensor CS2 is confirmed, the sub control module 16S may recognize that the first holder 130 is located at the reference position and may confirm that the needle N is withdrawn from the cannula C.

The first sensor CS1 and the second sensor CS2 may be provided as sensors that generate signals by contact, sensors that measure positions, or sensors that measure force applied within a predetermined range.

In one embodiment, the first sensor CS1 and the second sensor CS2 may include a conductivity material, and the first connection part of the main control module 16 and the second connection part of the sub control module 16S may also include a conductivity material. Accordingly, the first sensor CS1 may generate a signal by contacting the first connection part of the main control module 16, and the second sensor CS2 may generate a signal by contacting the second connection part of the sub control module 16S.

In another embodiment, the first sensor CS1 and the second sensor CS2, or the first connection part of the main control module 16 and the second connection part of the sub control module 16S may generate a signal when a predetermined force is applied. A signal may be generated when a force exceeding a preset threshold is applied to at least one of the first sensor CS1 and the first connection part of the main control module 16. In addition, a signal may be generated when a force exceeding a preset threshold is applied to at least one of the second sensor CS2 and the second connection part of the sub control module 16S.

As another embodiment, the first sensor CS1 and the second sensor CS2 may be sensors that measure distance. For example, the first sensor CS1 may measure a distance between the first sensor CS1 and the first connection part of the main control module 16 facing each other, and generate a signal based on a change in the distance. The second sensor CS2 may measure a distance between the second sensor CS2 and the second connection part of the sub control module 16S facing each other, and generate a signal based on a change in the distance. For example, the first sensor CS1 and the second sensor CS2 may be infrared sensors or ultrasonic sensors.

One end of the needle N is connected to the reservoir unit 200 so that the medical liquid may be delivered, and the other end is inserted into the cannula C and moved along the cannula C. Since the needle N is fixed to the first holder 130, it may be inserted into or released from the cannula C by the axial movement of the first holder 130.

Since the cannula C is fixed to the second holder 140, it may be inserted into the user's skin by moving the second holder 140 in the axial direction. Since the cannula C has a conduit shape that may accommodate the needle N, the medical liquid discharged from the needle N may be injected into the user. The cannula C remains inserted into the user's skin, but the needle N rises and separates from the object. However, the cannula C and the needle N form a path through which fluid moves, so that the medical liquid injected from the reservoir 210 may be injected into the user through the needle N and the cannula C.

The patch P is supported on the second body 14, and may fix the position of cannula C. Since the end of the cannula C is supported by the patch P, separation of the cannula C during storage or transportation may be prevented.

FIGS. 8 to 10 are cross-sectional views illustrating a driving operation of the needle assembly 100 of FIG. 5, and FIGS. 11 and 12 are cross-sectional views illustrating an operation in which the second holder 140 of FIG. 5 is mounted.

Referring to FIGS. 8 to 12, the driving of the needle assembly 100 is performed as follows. In the needle assembly 100, the elastic member 120 expands to move the first holder 130 and the second holder 140 to the insertion position when the sleeve 110 rotates, and the elastic member 120 contracts so that only the first holder 130 returns to the reference position when the sleeve 110 further rotates in the same direction. Thus, the needle N and the cannula C are inserted into the user's skin together, and then the needle N is withdrawn from the cannula C.

The needle assembly 100 is placed in its original position, that is, the position before the user uses the needle assembly 100. The moving protrusion 115 of the sleeve 110 is supported on the first stop groove PT₁, and the elastic member 120 maintains the compressed state between the knob 111 and the first holder 130. The first holder 130 and the second holder 140 are disposed above the needle assembly 100, so that the needle N and the cannula C are maintained inside the medical liquid injection device 1.

At this time, the first sensor CS1 is disposed at a position separated from the first connection part of the main control module 16, but the second sensor CS2 maintains contact with the second connection part of the sub control module 16S. However, since the medical liquid injection device 10 is in a state before being driven, an electrical signal may not be generated even if the second sensor CS2 contacts the second connection part of the sub control module 16S.

When the user rotates the knob 111 in one direction and the sleeve 110 rotates in one direction with respect to the first holder 130, the moving protrusion 115 moves beyond the first stop groove PT1. Then, as illustrated in FIG. 6, by the expansion of the elastic member 120 in the axial direction, the first holder 130 and the second holder 140 move downward, and the moving protrusion 115 may maintain contact with the first wall PT2.

When the first holder 130 and the second holder 140 move downward, the needle N and the cannula C are inserted into the user's skin. Since the first holder 130 exerts pressure on the second holder 140, the second holder 140 may pass through the first ledge 13a and the second ledge 13b. Since the second holder 140 is formed of the flexible material, when the first holder 130 presses the second holder 140, the first end 142 passes through the first ledge 13a, and the second end 143 passes through the second ledge 13b.

The cutout part is disposed on at least one of the first end 142 and the second end 143 of the second holder 140, so that the second holder 140 may be simply mounted on the first ledge 13a and the second ledge 13b. The cutout part allows deformation of the first end 142 and the second end 143 in the width direction, so that the position of the second holder 140 may be easily fixed.

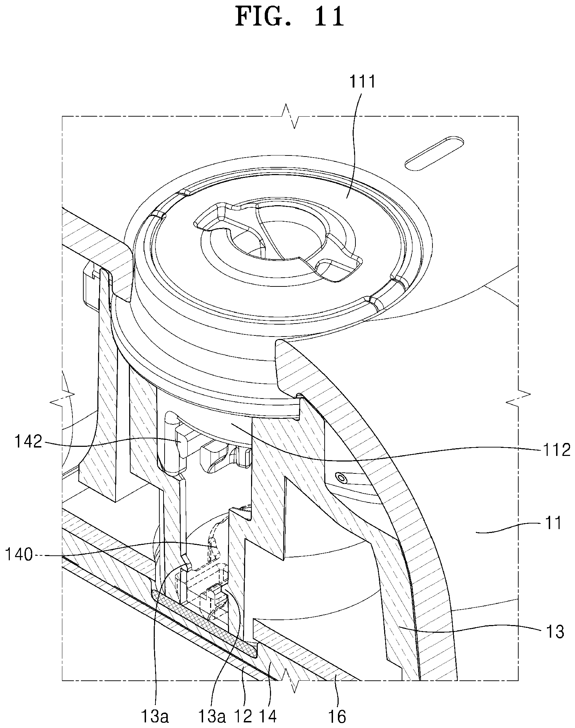

Referring to FIG. 11, the first cutout part 142d disposed at the first end 142 allows deformation of the first extension tab 142a in the width direction. When the first end 142 is inserted into the first ledge 13a, the first cutout part 142d deforms the first extension tab 142a in the width direction, so that the distance between the first locking protrusions 142b is reduced. The first end 142 may be fixed by passing the first locking protrusion 142b through the first ledge 13a.

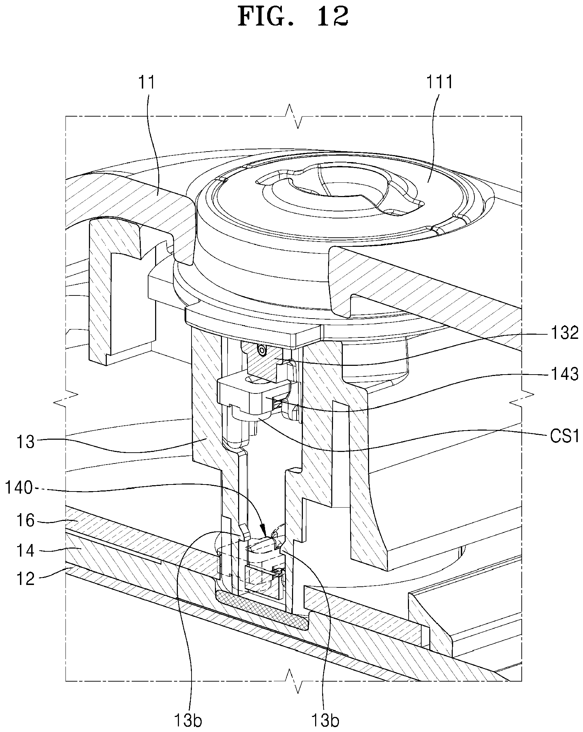

Referring to FIG. 12, the second cutout part 143d disposed at the second end 143 allows deformation of the second extension tab 143a in the width direction. When the second end 143 is inserted into the second ledge 13b, the second cutout part 143d deforms the second extension tab 143a in the width direction, so that the distance between the second locking protrusions 143b is reduced. The second locking protrusion 143b passes through the second ledge 13b so that the second end 143 may be fixed.

When the second extension tab 143a is deformed and the second end is mounted on the second ledge 13b, contact between the first sensor CS1 and the first connection part of the main control module 16 may be stably maintained. The first sensor CS1 may accurately measure whether the cannula C is inserted or the needle N is inserted.

At this time, the first sensor CS1 contacts the first connection part of the main control module 16. Since the first ledge 13a and the second ledge 13b fix the position of the second holder 140, the first sensor CS1 may stably maintain contact with the main control module 16.

In one embodiment, when the first sensor CS1 and the first connection part of the main control module 16 come into contact, an electrical signal may be generated, and the main control module 16 may sense that the needle N is advanced and the cannula C is normally inserted into the object through the electrical signal.

In another embodiment, even if the needle N advances and the cannula C is inserted into the object, electrical driving of the medical liquid injection device 10 does not proceed, and thus the electrical signal may not be generated in the main control module 16. Since the medical liquid injection device 10 is electrically driven when the needle N is withdrawn from the cannula C and the second sensor CS2 contacts the sub control module 16S, the electrical signal generated by the first sensor CS1 and the second sensor CS2 may be processed.

When the user further rotates the knob 111 in the one direction and the sleeve 110 rotates in the one direction with respect to the first holder 130, the moving protrusion 115 moves along the guide groove 134. When the inclined wall 115a of the moving protrusion 115 moves along the inclined surface of the second wall PT3, the first holder 130 rises again and the elastic member 120 is compressed again.

The rise of the second holder 140 is limited by the first ledge 13a and the second ledge 13b. The first ledge 13a supports the first end 142 and the second ledge 13b supports the second end 143, so even if the first holder 130 moves up and down, the second holder 140 does not move up and down. Upon the rise of the first holder 130, the needle N is withdrawn from the cannula C.

At this time, the second sensor CS2 contacts the second connection part of the sub control module 16S. When the first holder 130 returns to the reference position, the second sensor CS2 contacts with the second connection part of the sub control module 16S, so an electrical signal is generated. Since the sub control module 16S is electrically connected to the main control module 16, it is possible to recognize that the needle N has been withdrawn through the generated electrical signal.

As an example, if the first sensor CS1 and the second sensor CS2 generate electrical signals at the same time, the control module may recognize that the cannula C is inserted into the object and the needle N is withdrawn from the cannula C. In the main control module 16, when the electrical signal generated from the first sensor CS1 and the electrical signal generated from the second sensor CS2 and transmitted from the sub control module 16S are simultaneously measured, may recognize that the needle N is inserted into the object and then retreated to the reference position.

As an embodiment, when the electrical signals are generated from the first sensor CS1 and the second sensor CS2, the control module drives the driving module 300, so medical liquid injection may start.

Referring back to FIG. 3, the reservoir unit 200 is mounted on the first body 13 and the third body 15 and is connected to the needle assembly 100. In the reservoir unit 200, medical liquid D is stored in an inner space of the reservoir 210, and a fixed amount of medical liquid may be moved to the needle N according to the movement of a plunger.

The reservoir 210 is extended to a preset length in a longitudinal direction, and may store the medical liquid in the inner space. The reservoir 210 may discharge the medical liquid through the needle N by moving the plunger.

An inlet end of the reservoir 210 is connected to a lower portion of the medical liquid injection device 10, and the medical liquid may be injected by a medical liquid injector NI. The inlet end may have a first connection opening 212 connected to a guide groove 211.

An outlet end of the reservoir 210 is disposed spaced apart from the inlet end, and is connected to the needle N so that the medical liquid may be discharged. The outlet end may have a second connection opening 213 connected to the guide groove 211.

The guide groove 211 may be disposed on an inner surface of the reservoir 210. The guide groove 211 may be extended so that at least some sections connect between the inlet end and the outlet end.

The guide groove 211 may form a path for guiding gas remaining in the inner space of the reservoir 210 to the needle assembly 100. Inside the reservoir 210, more specifically, in a gap between a front end of the reservoir 210 and the plunger, there is gas (air) remaining during assembly or manufacturing. When the medical liquid is injected into the reservoir 210 through the external medical liquid injector NI, the gas may be discharged through the needle N along the guide groove 211.

The driving module 300 may generate driving force and transmit the driving force to the driving unit 400. The driving force transmitted by the driving unit 400 linearly moves the plunger inside the reservoir to discharge the medical liquid.

When the driving units 400 are engaged with each other by the clutch unit 500, the driving module 300 rotates a driving wheel of the driving unit 400, and the rotation of the driving wheel moves a rod linearly so that the plunger may move.

The driving module 300 may be used with all types of devices having medical liquid suction power and medical liquid discharge power by electricity. For example, all kinds of pumps such as mechanical displacement type micropumps and electromagnetic motion type micropumps may be used. The mechanical displacement type micropump is a pump that uses the motion of a solid or fluid, such as a gear or diaphragm, to generate a pressure difference to induce the flow of fluid, and includes a diaphragm displacement pump, a fluid displacement pump, a rotary pump, and the like. The electromagnetic motion type micropump is a pump that directly uses electrical or magnetic energy to move a fluid, and includes an electro hydrodynamic pump (EHD), an electro osmotic pump, a magneto hydrodynamic pump, an electro wetting pump, and the like.

The battery 350 may supply electricity to the medical liquid injection device 10 to activate each part. In the drawing, a pair of batteries 350 are illustrated, but are not limited thereto, and may be set in various ways according to the capacity, use range, and use time of the medical liquid injection device 10.

The battery 350 is disposed adjacent to the driving unit 400 and may supply electricity to the driving unit 400. In addition, the battery 350 is connected to the main control module 16, and the sensor unit may measure data on the number of rotations or rotational speed of the driving unit 400, the amount of medical liquid stored in the reservoir 210, amount of medical liquid injected to the user, etc. based on the measured electrical signal.

The driving unit 400 may be installed between the driving module 300 and the reservoir unit 200 to move the plunger disposed in the reservoir 210 with the driving force generated by the driving module 300.

The trigger member 600 may generate a mechanical signal through which the medical liquid of the medical liquid injection device 10 is injected. The trigger member 600 is rotatably disposed on one side of the third body 15, the trigger member 600 rotates to start driving the driving module 300, and at the same time, the clutch unit may drive and connect the driving unit 400.

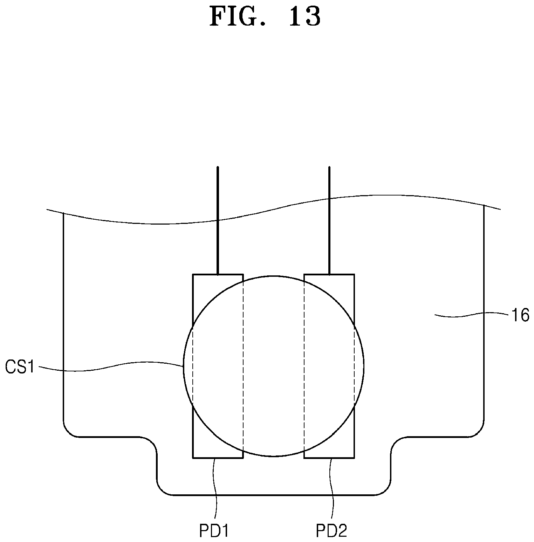

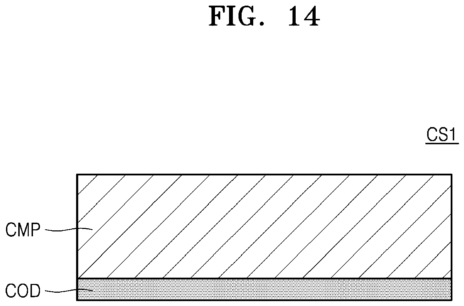

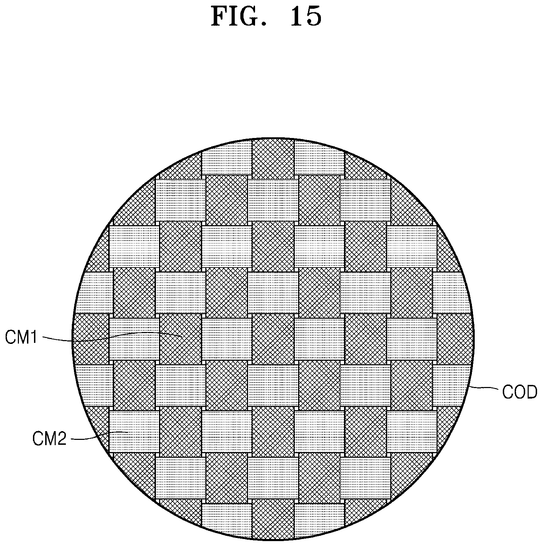

FIG. 13 is a diagram illustrating an arrangement of sensing unit and connection part, FIG. 14 is a cross-sectional view of the sensing unit of FIG. 13, and FIG. 15 is a plan view illustrating a contact unit of FIG. 14.

The sensing unit is placed on one side of the holder unit HU and may move in a height direction according to the movement of the needle N. The sensing unit may measure the position of the needle N in the height direction.

The sensing unit may include the first sensor CS1 which is disposed on one side of the holder unit HU and senses whether the needle N is located at the insertion position where the needle N is inserted into the object. In addition, the sensing unit may include the second sensor CS2 which is disposed on the other side of the holder unit HU and senses whether the needle N is located at the reference position that is the position before the needle N is inserted into the object.

The sensing unit may include at least one of the aforementioned first sensor CS1 and second sensor CS2, but hereinafter, for convenience of description, the first sensor CS1 will be mainly described. Structure and arrangement of the first sensor CS1 described later may also be applied to the second sensor CS2.

A connection part is arranged to face the sensing unit and may come into contact with the sensing unit according to the movement of the needle N. The connection part may be connected to the control module. The connection part includes a pair of pads, and the pair of pads may be electrically connected by contact of the sensing unit.

The connection part may include the first connection part disposed in the main control module 16 and the second connection part disposed in the sub control module 16S. Hereinafter, for convenience of description, the first connection part disposed in the main control module 16 will be mainly described. That is, the first connection part applied to the main control module 16 may also be applied to the second connection part of the sub control module 16S.

Referring to FIGS. 13 to 15, the first sensor CS1 may include a base CMP and a contact unit COD.

One side of the base CMP may be supported on the holder unit HU. The base CMP may be installed in the second holder 140, and may be specifically mounted in the second seating groove 143f of the second holder 140.

The base CMP may have a predetermined height, and in particular, may have a height greater than that of the contact unit COD. The base CMP may have a preset height so that the first sensor CS1 is compressed when it contacts the connection part.

The base CMP may be formed of a shrinkable material. The base CMP may be formed of a material that is compressed by an external force and whose shape is restored when the external force is released. The material of the base CMP is not limited to a specific material, and may be formed of, for example, a sponge.

The contact unit COD is placed on the other side of the base CMP and may have conductivity. The contact unit COD may be arranged to face the connection part. That is, the contact unit COD is disposed to face a first pad PD1 and a second pad PD2, and the contact unit COD may electrically connect the first pad PD1 and the second pad PD2 when the first sensor CS1 descends. Therefore, when the contact unit COD contacts the connection part, the base CMP may be compressed in the height direction.

In one embodiment, the contact unit COD may have a thin layer having conductivity. The contact unit COD may be placed on an outer surface of the base CMP as a thin plate.

In one embodiment, the contact unit COD may be woven such that a first conductive member CM1 and a second conductive member CM2 cross each other (see FIG. 15).

At least one of the first conductive member CM1 and the second conductive member CM2 may have conductivity on the outside. For example, the first conductive member CM1 and the second conductive member CM2 may be formed of wires or bands, and at least one of the first conductive member CM1 and the second conductive member CM2 may have conductivity.

The first conductive member CM1 and the second conductive member CM2 may be coated with a conductive material on the outside or formed of a conductive material themselves.

Since the first conductive member CM1 and the second conductive member CM2 are woven to form the contact unit COD, the contact unit COD may be deformed according to the shape deformation of the base CMP. When the first sensor CS1 contacts the connection part, the shape of the base CMP is deformed, and the contact unit COD may also be deformed according to the shape deformation of the base CMP. When the contact unit COD contacts the first pad PD1 and the second pad PD2, its shape is deformed, so the first sensor CS1 and the connection part are in stable contact and electrical connectivity is strengthened, so that the positions of the needle N and the holder unit HU may be accurately measured.

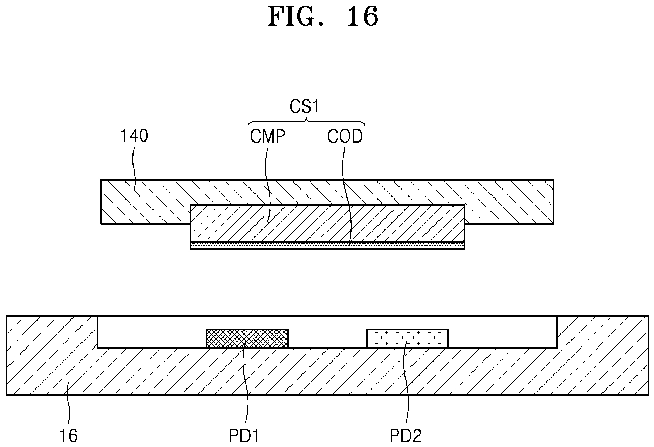

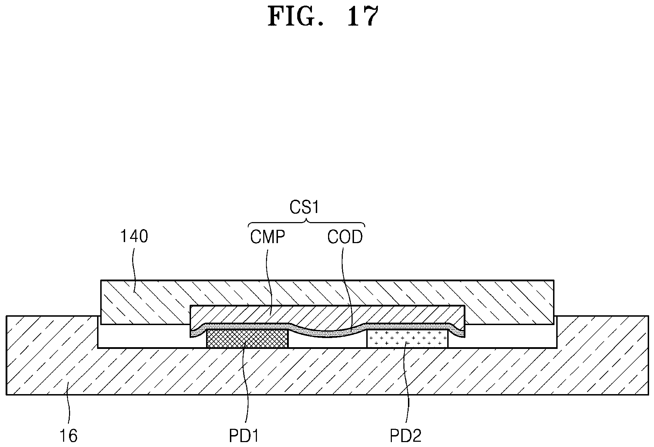

FIGS. 16 and 17 are diagrams illustrating contact between the sensing unit and the connection part of FIG. 13.

Referring to FIGS. 16 and 17, since the shape of the base is deformed, the sensing unit may stably maintain contact with the connection part.

The connection part may be installed in the control module. The control module includes the circuit board, and the first pad PD1 and the second pad PD2 may be installed on the circuit board.

For example, the connection part may be formed by patterning the first pad PD1 and the second pad PD2 on the circuit board. The first pad PD1 and the second pad PD2 are spaced apart from each other.

As another example, after patterning the first pad PD1 and the second pad PD2 on the circuit board, outer sides of the first pad PD1 and the second pad PD2 may be removed from the circuit board by a predetermined thickness. Accordingly, the first pad PD1 and the second pad PD2 of the connection part may have a protruding shape. Since the first pad PD1 and the second pad PD2 protrude from a surface, contact with the first sensor CS1 may be maintained more firmly and stably.

Referring to FIGS. 8 and 16, before the needle N is inserted into the object, the first sensor CS1 is separated from the connection part, and the first pad PD1 and the second pad PD2 are not electrically connected.

Referring to FIGS. 9 and 17, when the needle N is inserted into the object, the first sensor CS1 contacts the connection part. Since the contact unit COD of the first sensor CS1 contacts the first pad PD1 and the second pad PD2, the first pad PD1 and the second pad PD2 are electrically connected, and the generated electrical signal is transmitted to the controller, it may be recognized that the needle N is inserted into the object.

Since the base CMP of the first sensor CS1 is made of the shrinkable material, the base CMP is compressed in the height direction when the first sensor CS1 contacts the connection part. In detail, in the base CMP, parts corresponding to the first pad PD1 and the second pad PD2 are greatly compressed, and the rest are compressed relatively little. As a result, the first pad PD1 and the second pad PD2 may stably maintain contact with the contact unit COD.

The contact unit COD may be formed by weaving the first conductive member CM1 and the second conductive material CM2. Since the first conductive member CM1 and the second conductive material CM2 are woven, the contact unit COD may obtain flexibility. Therefore, when the base CMP is compressed by the descent of the first sensor CS1, the contact unit COD may be curved along with the shape change of the base CMP. The contact unit COD may accurately measure the position of the needle N because the first pad PD1 and the second pad PD2 are in stable contact.

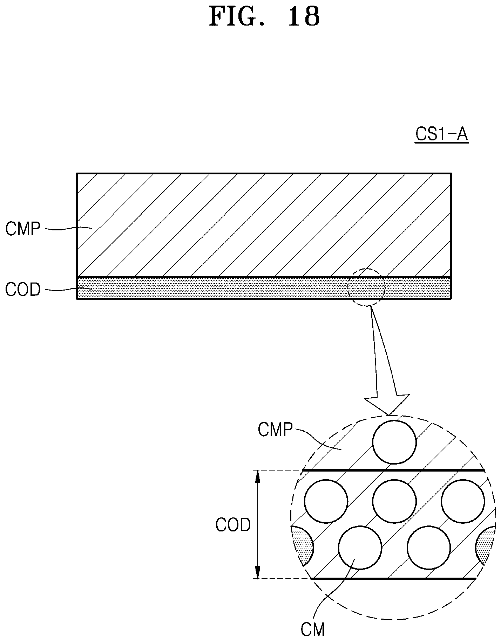

FIGS. 18 to 20 are cross-sectional views illustrating another embodiment of the sensing unit of FIG. 14.

Referring to FIG. 18, a first sensor CS1-A may include a base CMP and a contact unit COD.

The base CMP may be formed of a porous material, and may have compressibility and resilience due to the porosity.

The contact unit COD is defined as an outer area of the base CMP, and may be formed by filling empty spaces inside with a conductive material CM. That is, the contact unit COD may be formed by filling small holes of the base CMP with the conductive material CM. The contact unit COD sprays the conductive material CM on an outer surface of the base CMP, and the conductive material CM may fill the empty spaces inside.

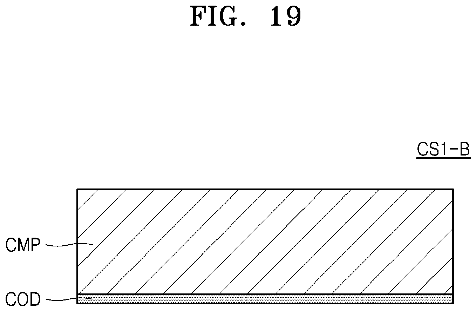

Referring to FIG. 19, a first sensor CS1-B may include a base CMP and a contact unit COD.

The base CMP may have a predetermined height, compressibility, and shape resilience. The contact unit COD may have a thin plate structure and may be attached to an outer surface of the base CMP.

In one embodiment, the contact unit COD may have a preset roughness on a surface exposed to an outside. Since the surface of the contact unit COD has the preset roughness, a frictional force is formed between the first pad PD1 and the second pad PD2 and the contact unit COD, and the first sensor CS1-B and the connection part may maintain stable contact.

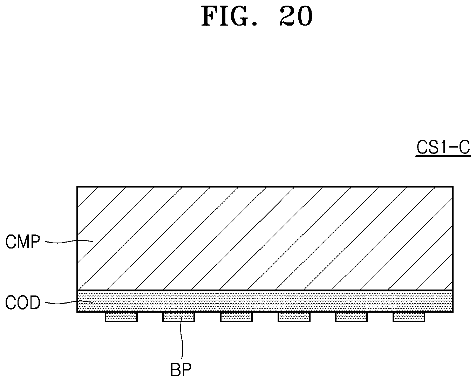

Referring to FIG. 20, a first sensor CS1-C may include a base CMP and a contact unit COD.

The base CMP may have a predetermined height, compressibility, and shape resilience. The contact unit COD is disposed on an outer surface of the base CMP and may have a plurality of protrusions BP.

The contact unit COD may have the protrusion BP on a surface exposed to an outside. The protrusion BP may contact the first pad PD1 or the second pad PD2 to electrically connect the first pad PD1 and the second pad PD2.

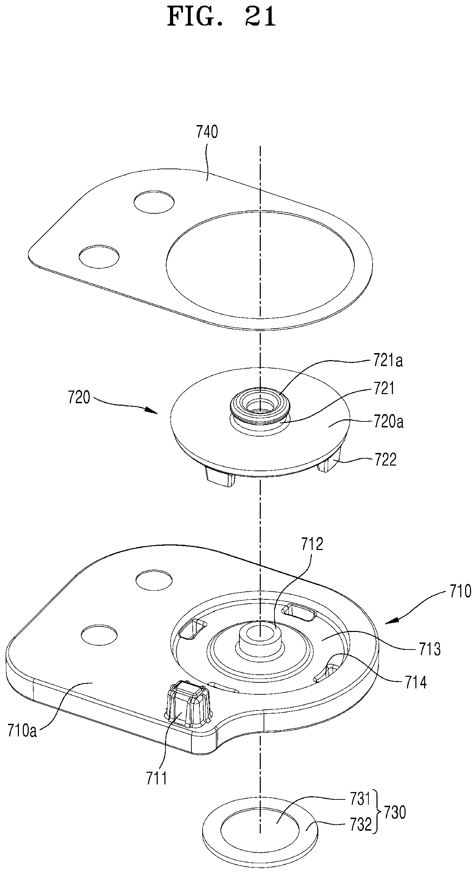

FIG. 21 is an exploded perspective view illustrating a needle cover assembly 700 of FIG. 3.

Referring to FIGS. 4 and 21, the needle cover assembly 700 may be mounted under the needle assembly 100. The needle cover assembly 700 may prime air stored in the reservoir unit 200 before injecting the medical liquid. When the medical liquid is injected into the reservoir 210 through the medical liquid injector NI, the gas (air) remaining in the reservoir 210 may be discharged to the outside.

The needle cover assembly 700 may have a first cover 710, a second cover 720, a filter member 730 and an adhesive layer 740.

The first cover 710 may be disposed on a lower portion of the medical liquid injection device 10. The second cover 720 may be inserted into an opening of the first cover 710 and assembled.

The first cover 710 may include an insertion protrusion 711 protruding from a first block 710a. The insertion protrusion 711 is inserted into an insertion groove of the second body 14 to fix the needle cover assembly 700 to the base body.

The first cover 710 may include an insertion groove 713 into which the second cover 720 is inserted, and a duct 712 disposed at a center of the insertion groove 713. In addition, a plurality of fixing grooves 714 into which a fixing protrusion 722 of the second cover 720 are inserted may be disposed in the insertion groove 713.

The second cover 720 is assembled to the first cover 710, and the needle N and/or the cannula C may be aligned at an center thereof. The second cover 720 may have a storage space penetrating in the center in a height direction and storing the medical liquid D.

The first cover 710 is stronger than the second cover 720. The first cover 710 is a part that is exposed to the outside and is made of a material with rather high rigidity. The second cover 720 is assembled to the first cover 710 and is formed of a material with less rigidity than the first cover 710 in order to be inserted into an opening of the second body 14.

A protrusion 721 inserted into the second body 14 may be provided at a center of a second block 720a of the second cover 720 . The protrusion 721 may have an extension end 721a whose end extends outward in a radial direction. The extension end 721a may be inserted into the second body 14 so that the second cover 720 may be fixed to the lower portion of the medical liquid injection device 10. Since the protrusion 721 and the extension end 721a have certain flexibility, they may be inserted into the second body 14 with an interference fit.

The second cover 720 includes the fixing protrusion 722, and the fixing protrusion 722 is inserted into the first cover 710, so that the first cover 710 and the second cover 720 may be assembled.

The protrusion 721 of the second cover 720 is inserted into an opening at a bottom of the second body 14. A diameter G2 of the protrusion 721 is set slightly larger than a diameter G1 of the opening. Since the second cover 720 has a predetermined elasticity, the protrusion 721 may be inserted into and fixed to the opening.

The second cover 720 has an inner diameter G3, so the needle N and the cannula C may be aligned at a top thereof. The inner diameter G3 forms the storage space of the second cover 720, and the medical liquid may be stored or the gas may be moved and discharged. The duct 712 of the first cover 710 also has the inner diameter G3, so that the medical liquid may be stored in an inner space of the duct 712 or the gas may be moved and discharged.

The filter member 730 is mounted on the second cover 720. The filter member 730 is disposed below the storage space of the second cover 720, and the gas such as the air passes through the filter member 730, but liquid such as the medical liquid does not pass through the filter member 730. Thus, the air discharged from the needle N passes through the filter member 730 and is discharged to the outside, but the medical liquid discharged from the needle may be stored in a storage space defined by the second cover 720 and the filter member 730.

The shape of the filter member 730 may change according to the amount of the medical liquid stored in the storage space. For example, when the medical liquid is filled in the storage space, the filter member 730 expands downward, so that the user may recognize that the medical liquid has flowed into the needle cover assembly 700.

The adhesive layer 740 is disposed on one side of the needle cover assembly 700, and may attach the needle cover assembly 700 to the attachment part 12.

The alarm unit 800 is disposed inside or outside the medical liquid injection device 10, and may notify the user of normal operation or malfunction of the medical liquid injection device 10.

For example, the alarm unit 800 is disposed below the housing 11 and is connected to the circuit board. The alarm unit 800 may deliver an alarm to an external user by generating a warning sound or generating a light.

In the needle assembly 100 and the medical liquid injection device 10 according to the present disclosure, the cannula C may be stably inserted and fixed to the object. When the first holder 130 moves downward, the second holder 140 also moves downward, inserting the cannula C and the needle N into the object, and since the position of the second holder 140 may be fixed, the medical liquid is stably injected into the object.

The needle assembly and medical liquid injection device according to the present disclosure may safely and accurately sense whether the needle is inserted or whether the needle is withdrawn. When the needle is inserted into or withdrawn from the object, a sensor unit may accurately sense the position of the needle by contacting a connection part. In particular, the sensor unit is formed of a material having compressibility and shape restoring force, so that when the sensor unit contacts a pad of the connection part, the sensor unit may be stably contacted with the connection part by being compressed.

The needle assembly 100 and the medical liquid injection device 10 according to the present disclosure may accurately measure the movement and position of the needle N. The first sensor CS1 measures the motion of the needle N inserted into the object, and the second sensor CS2 measures the motion of the needle N retracting to the reference position.

The needle assembly 100 and the medical liquid injection device 10 according to the present disclosure may measure that the cannula C is inserted into the object and the needle N is withdrawn from the cannula C. The first sensor CS1 measures that the cannula C is inserted into the object, and the second sensor CS2 measures that the needle N is withdrawn from the cannula C and moved to the position where the medical liquid may be delivered to the cannula C. When the first sensor CS1 and the second sensor CS2 come into contact at the same time, it may be confirmed that the medical liquid injection device 10 is ready to inject the medical liquid into the object.

In the needle assembly 100 and the medical liquid injection device 10 according to the present disclosure, the cannula C may be simply inserted into the object. The second holder 140 includes the first end 142 and the second end 143 extending to both sides, and since at least one of the first end 142 and the second end 143 is partially removed, the deformation of the second holder 140 may be allowed. Since the cutout part allows the deformations in the width direction of the first end 142 and the second end 143 when the second holder 140 passes through the ledge, the second holder 140 may simply pass through the ledge and be fixed in position by the ledge.

The spirit of the present disclosure should not be limited to the above-described embodiments and should not be determined, and not only the claims to be described later, but also all ranges that are equivalent to or equivalently changed from the claims fall within the scope of the spirit of the present disclosure. would be said to belong.

### INDUSTRIAL APPLICABILITY

According to one embodiment of the present disclosure, a medical liquid injection device may be applied to various industrially available devices. The medical liquid injection device may be applied to devices for delivering various medical liquids.

## Claims

1. A needle assembly comprising:
a needle;
a cannula into which the needle is inserted;
a first holder supporting the needle;
a sleeve in which the first holder is disposed in an inner space thereof; and
a second holder supporting the cannula, disposed to face one side of the first holder, and having a first end and a second end which extend outward,
wherein at least one of the first end and the second end has
an cutout part with a part removed by a preset length.

2. The needle assembly of claim 1,
wherein at least one of the first end and the second end has
an end extending to protrude from an outer circumferential surface of the first holder, and a part of the cutout part is disposed outside the outer circumferential surface of the first holder in a radial direction.

3. The needle assembly of claim 2,
wherein another part of the cutout part is disposed between an inner circumferential surface and the outer circumferential surface of the first holder.

4. The needle assembly of claim 1,
wherein the first end extends to one side and has a first cutout part having a slit shape with an open end.

5. The needle assembly of claim 4,
wherein the second end extends to the other side and has a second cutout part extending in a longitudinal direction and having an opening with a closed end.

6. The needle assembly of claim 1,
wherein the second holder is formed such that
the second end is disposed at a higher position than the first end, and a sensor is disposed at an end of the second end.

7. A medical liquid injection device comprising:
a base body;
a housing disposed above the base body;
a sleeve mounted to the base body and the housing;
a first holder inserted into an inner space of the sleeve and supporting a needle; and
a second holder supporting a cannula into which the needle is inserted, disposed to face one side of the first holder, and having a first end and a second end extending outward,
wherein at least one of the first end and the second end has
a cutout part with a part removed by a preset length.

8. The medical liquid injection device of claim 7,
wherein at least one of the first end and the second end has
an end extending to protrude from an outer circumferential surface of the first holder, and a part of the cutout part is disposed outside the outer circumferential surface of the first holder in a radial direction.

9. The medical liquid injection device of claim 7,
wherein at least one of the first end and the second end has a locking protrusion protruding from an outside of the cutout part, and
the base body has a ledge supporting the locking protrusion when the second holder descends.

10. The medical liquid injection device of claim 7,
wherein the first end extends to one side and has a first cutout part with a slit shape with an open end.

11. The medical liquid injection device of claim 10,
wherein the second end extends to the other side and has a second cutout part extending in a longitudinal direction and having an opening with a closed end.

12. The medical liquid injection device of claim 7,
wherein the second holder is formed such that the second end is disposed at a higher position than the first end, a sensor is disposed at an end of the second end, and
the sensor contacts one side of the base body when the second holder descends.
